# EUROPÄISCHE PATENTSCHRIFT

(11) **EP 0 542 098 B1**
(45) Veröffentlichungstag und Bekanntmachung des Hinweises auf die Patenterteilung: **13.06.2001**
(21) Anmeldenummer: 92118795.1
(22) Anmeldetag: 03.11.1992
(51) Int. Cl.: C07D 233/76, C07D 233/78, C07D 233/74, C12P 41/00, C12P 13/04

(54) **Verfahren zur chemischen Racemisierung von 5-monosubstituierten Hydantoinen**
Process of chemical racemisation of 5-monosubstituted hydantoins
Procédé de racémisation d'hydantoines 5-substituées

(30) Priorität: 15.11.1991 DE 4137581
(43) Veröffentlichungstag der Anmeldung: 19.05.1993
(73) Patentinhaber: Degussa AG, 40474 Düsseldorf (DE)
(72) Erfinder: Wagner, Fritz, Prof. Dr., W-3300 Braunschweig (DE); Pietzsch, Markus, W-3300 Braunschweig (DE); Syldatk, Christoph, Dr., W-3200 Hildesheim (DE)

(56) Entgegenhaltungen:
- WO-A-91/08196
- JOURNAL OF ORGANIC CHEMISTRY Bd. 55, 1990, EASTON US Seiten 4755 - 4757 R.A. LAZARUS.
- CHEMICAL ABSTRACTS, vol. 85, no. 25, 20. Dezember 1976, Columbus, Ohio, US; abstract no. 192096k, K.H. DUDLEY ET AL.
- DATABASE WPIL Week 9126, Derwent Publications Ltd., London, GB; AN 91-188025
- AGRICULTURAL AND BIOLOGICAL CHEMISTRY Bd. 51, Nr. 3, 1987, TOKYO JP Seiten 721 - 728 K. YOKOZEKI AND K. KUBOTA.
- G.C. BARRETT. ''amino acids and peptides'' 1989
- JOURNAL OF BIOTECHNOLOGY Bd. 14, 1990, AMSTERDAM NL Seiten 345 - 362 C. SYLDATK ET AL.

## Beschreibung

Die Erfindung betrifft ein Verfahren zur Racemisierung von 5-monosubstituierten Hydantoinen.

Die bei der chemischen Synthese von Aminosäuren entstehenden Racemate werden meist durch enzymatische Spaltung in die einzelnen Enantiomere getrennt. Dies kann z. 8. durch stereospezifische enzymatische Spaltung der N-acylierten Aminosäuren oder durch enantioselektive enzymatische Spaltung der entsprechenden in 5-Stellung monosubstituierten Hydantoinen erfolgen, wobei jeweils ein ungespaltenes Enantiomer der Ausgangsverbindung abgetrennt und nach einem Racemisierungsprozeß der enzymatischen Spaltung erneut unterworfen werden kann. Einen guten Überblick über mögliche Racemisierungen gibt E. Adams in P. D. Boyer, "The Enzymes", Vol. VI, New York, 1972, 479 - 507 (Amino Acid Racemases and Epimerases). Prinzipiell kann der Racemisierungsprozeß dabei auf chemischem, physikalisch-chemischem oder enzymatischem Wege erfolgen, wobei je nach Prozeßbedingungen auch kombinierte Verfahren möglich sind. Eine enzymatische Hydantoinracemisierung wird von M. Pietzsch in "Stereochemische Untersuchungen zur enzymatischen Hydrolyse von D,L-5-monosubstituierten Hydantoinen", Diplomarbeit, TU Braunschweig, 1989 beschrieben, wobei eine Racemase aus Arthrobacter spec. (DSM 3747) Verwendung fand. Enzymatische Verfahren haben jedoch den Nachteil, daB die bekannten Racemasen eine hohe Substratspezifität besitzen und deshalb nur für sehr spezielle Verfahren eingesetzt werden können. Für viele Hydantoine sind noch keine Racemasen bekannt.

Aus diesem Grund überwiegt bei den Hydantoinen wie auch Aminosäuren die chemische oder physikalisch-chemische Racemisierung, bei der die Edukte meist in einem basischen Milieu einem Temperatur-Zeit-Programm unterworfen werden. Für Aminosäuren sind auch Verfahren bekannt, bei denen die Racemisierung an stark basischen Anionenaustauschern durchgeführt wird, wobei relativ niedrige Temperaturen (25 - 45°C) ausreichend sind. Diese Anionenaustauscher-Verfahren gelingen jedoch nur bei neutralen oder basischen Aminosäuren und erfordern die Verwendung von Kupfer(II)-ionen, wobei als Zwischenstufe ein reaktiver Kupfer(II)-Schiff sche Base-Komplex entsteht. Ein Überblick über diese Verfahren gibt G. C. Barrett, Amino Acids and Peptides 20, 1 - 51 (1989), spezielle Verfahren sind beschrieben in Makromol. Chem. 187, 1065 - 1076 (1986). Diese Verfahren sind äußerst aufwendig und nur im Labormaßstab durchzuführen, da neben den Kupferionen auch ein mit 4-Hydroxy-3-formylbenzolsulfonsäure (5-Sulfosalicylaldehyd) neutralisierter Ionenaustauscher zur Anwendung kommt, der einerseits eine Regeneration erfordert und andererseits eine aufwendige Reinigung der erhaltenen Produkte, da insbesondere die Kupferionen quantitativ entfernt werden müssen. Außerdem hat die Verwendung von Übergangsmetallkationen den Nachteil, daß unter Umständen sehr stabile Komplexe mit den verwendeten Aminosäuren gebildet werden können.

Die chemische Racemisierung von 5-monosubstituierten Hydantoinen verläuft über die Keto-Enol-Tautomerie (Chem. Rev. 46, 403 - 470 (1950)) und wird, wie schon sehr früh festgestellt, durch Basen katalysiert (Am. Chem. J. 44, 48 - 60 (1910). Neuere Untersuchungen über die chemische Racemisierung von Hydantoinen wurden nur mit speziellen Substraten durchgeführt, z. B. 5-(p-Hydroxyphenyl)-hydantoin in Agric. Biol. Chem. 51, 721 - 728 (1987) und 5-Benzylhydantoin in J. Org. Chem. 55, 4755 - 57 (1990). Die Untersuchungen zeigen, daß nur beim 5-(p-Hydroxyphenyl)-hydantoin eine schnelle Racemisierung aufgrund der Mesomeriestabilisierung durch den 5-Substituenten (50 % nach 20 min.) eintritt, alle anderen Hydantoine racemisieren erst nach mehreren Stunden.

Aufgabe der vorliegenden Erfindung ist daher ein Verfahren zur Racemisierung von 5-monosubstituierten Hydantoinen, wobei die Racemisierung unter milden Bedingungen durchführbar und mit einer Vielzahl von Substraten möglich sein soll. Das Verfahren soll außerdem möglichst einfach in seiner Durchführbarkeit sein, d. h. auf problematische Fremdionen, wie z. B. verschiedene Übergangsmetallkationen oder zusätzliche Verbindungen, die aufwendig zu entfernen sind, sollte verzichtet werden können. Die Racemisierung soll dabei in einer annehmbar kurzen Zeit und unter guten Ausbeuten stattfinden.

Gelöst wird diese Aufgabe hinsichtlich des Verfahrens durch ein Racemisierungsverfahren, bei dem das Enantiomer eines in 5-Stellung substituierten Hydantoins in Anwesenheit eines Anionenaustauschers bei einem pH im Bereich 6 - 13,5 umgesetzt wird.

Die eingesetzten Hydantoine haben die allgemeine Formel 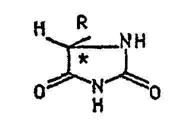 worin
- *: ein Symmetriezentrum bedeutet, an dem die Racemisierung stattfindet, und
- R: ein Rest einer Aminosäure ist, wobei die Aminosäure proteinogen oder nicht proteinogen sein kann.

Typische Reste R sind

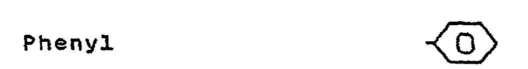

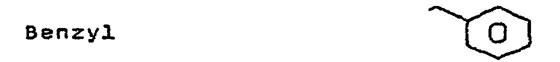

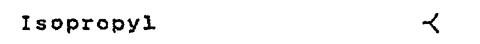

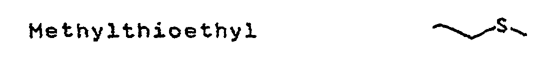

Der Ionenaustauscher kann in Gelform, als Festkörper oder auch in flüssiger bzw. löslicher Form vorliegen. Gelförmige- und Festkörperionenaustauscher können dabei z. B. in Festbettreaktoren eingesetzt werden, flüssige bzw. lösliche Ionenaustauscher kommen meist z. B. in durchmischten Reaktoren zur Anwendung. Neben den Festbettreaktoren (z. B. Chromatographiesäulen) sind die gelförmigen und festen Ionenaustauscher auch in durchmischten Reaktoren, wie z. B. Wirbelschicht-, gerührte- und Membranreaktoren einsetzbar.

Geeignet ist hierbei die Verwendung von Anionenaustauschern in einem pH-Bereich von 6,0 - 13,5, bevorzugt 6,5 - 11, wobei insbesondere stark basische Anionaustauscher, z. B. solche mit quartären Ammoniumgruppen oder auf Phosphoniumbasis besonders gute Ergebnisse zeigen. Ist der pH zu niedrig, erfolgt keine Umsetzung; bei zu hohem pH racemisiert das Hydantoin auch ohne der Gegenwart des Ionenaustauschers. Bevorzugt wegen ihrer geringen Kosten sind dabei Ionenaustauscher mit zumindest teilweise organischem, vorzugsweise rein organischem Aufbau.

Besonders günstig ist, daß erfindungsgemäß das zu racemisierende Hydantoin in einer Pufferlösung mit dem Ionenaustauscher in Kontakt gebracht werden kann, so daß Nebenreaktionen weitgehend vermieden werden können. Mit dem Puffersystem kann insbesondere bei der Verwendung eines Festkörperionenaustauschers ggf. auch ein gewünschter pH eingestellt werden.

Die Racemisierung erfordert bei handelsüblichen Ionenaustauschern meist eine Kontaktzeit von Substrat und Ionenaustauscher zwischen 5 min und 2 h pro ml Ionenaustauscher bei einer Temperatur zwischen ca. 0 und ca. 100 °C (Temperierbereich bei wässrigen Lösungen), vorzugsweise 20 - 70 °C und einer Konzentration von 0,1 - 250 g/l, vorzugsweise 0,2 - 50 g/l Hydantoin in Wasser bzw. dem Puffersystem.

Das erfindungsgemäße Verfahren hat bei seiner verblüffenden einfachen Durchführbarkeit die überzeugenden Vorteile, daß mit einer einzigen Ionenaustauscherart eine Vielzahl unterschiedlicher Hydantoine racemisiert werden kann, die in 5-Position aliphatische, geladene oder anders funktionalisierte und/oder aromatische Substituenten enthalten. Nur bei einem Wechsel der Lösung sollte der Ionenaustauscher vorher mit dem Puffersystem erneut äquilibriert werden, eine Regeneration ist i. d. R. nicht
erforderlich. Das erfindungsgemäße Verfahren eignet sich dabei gleichermaßen für die Racemisierung von D-5- und L-5-substituierten Hydantoinen, so daß das Verfahren bei der Produktion von 0- und L-*α*-Aminosäuren Einsatz finden kann; Durch geeignete Wahl der Parameter (Puffersystem, pH, Ionenaustauschmaterial, Temperatur, Durchflußgeschwindigkeit) kann praktisch jedes eingesetzte Hydantoin vollständig racemisiert werden.

Das Puffersystem ist günstigerweise in einer Konzentration zwischen 0,005 und 1,0 mol/l, vorteilhaft sind Konzentrationen zwischen 0,01 und 0,1 mol/l. Geeignete Systeme sind z. B. Glycin (NaOH). Phosphat (K⁺, Na⁺), Tris (HCl) (Tris(hydroxymethyl)-aminomethan), besonders gute Umsätze gibt Glycin / NaOH.

Prinzipiell kann auch ohne einem zusätzlichen Puffersystem verfahren werden, wobei gegebenenfalls der pH durch Zugabe von Säure (z. B. HCl) oder Base (z. B. NaOH) direkt zum zu racemisierenden Hydantoin eingestellt werden kann. Auch mit dem zu racemisierenden Hydantoin anfallende Begleitstoffe können in ein Puffersystem mit einbezogen werden.

Nicht funktionalisiertes Gelmaterial sowie Kationenenaustauscher sind als Katalysator ungeeignet (z. B. Sephacryl S-300, ein Gelfiltrationsmedium bzw. Amberlite IR-122, ein stark saurer Kationenaustauscher), bedingt geeignet sind schwache Ionenaustauscher (z. B. vom DEAE-Typ, z. B. DEAE-Sepharose) und stark abgeschirmte (flüssige) Anionenaustauscher (z. B. Tetrabutylammoniumhydrogensulfat), da diese für einen vergleichbaren Umsatz wesentlich größere Verweilzeiten des Substrates auf der Säule erfordern, als starke Ionenaustauscher (z. B. Ionenaustauscher vom Q-Typ, z. B. Q-Sepharose von Pharmacia, Freiburg oder Amberlite IRA-410 (hydrophobe Polystyrolmatrix)).

Wird im basischen Milieu racemisiert, so steigt die Racemisierungsgeschwindigkeit mit dem pH, wobei dem oberen pH-Wert durch das Säulenmaterial und durch die Stabilität des Hydantoins Grenzen gesetzt sind. Auch erhöhte Temperaturen beschleunigen die Racemisierungsgeschwindigkeit. Weiterhin wird die Racemisierungsgeschwindigkeit durch die Molarität des verwendeten Puffersystems und die Pufferart beeinflußt.

Das erfindungsgemäße Verfahren wird anhand von Figuren sowie der nachfolgenden Beispiele näher erläutert:

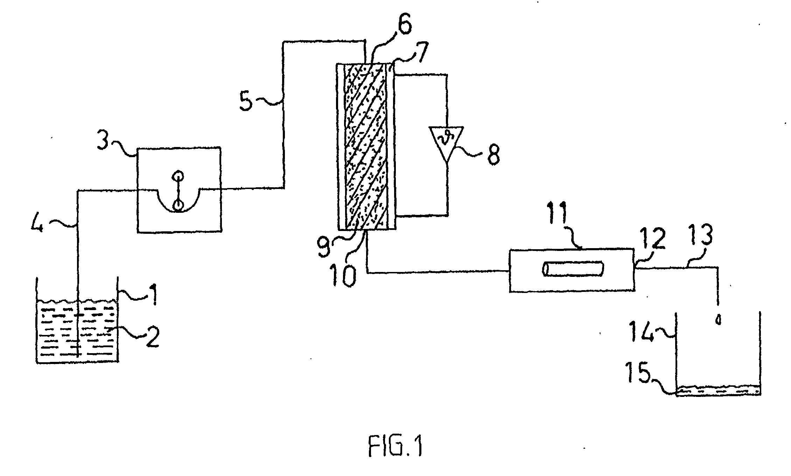

- Fig. 1: zeigt schematisch einen Aufbau zur Durchführung des erfindungsgemäßen Verfahrens,
- Fig. 2: die Racemisierung von D-5-Indolylmethylhydantoin (D-5-IMH) an Q-Sepharose in Abhängigkeit von der Flußrate. und
- Fig. 3: die Racemisierung von D-5-IMH an Amberlite IRA 410 in Abhängigkeit von der Flußrate.

In einer Vorlage 1 befindet sich eine Lösung 2 des Puffersystems und des zu racemisierenden Hydantoins. Eine Schlauchpumpe 3 saugt über eine Leitung 4 die Lösung 2 an und pumpt diese über eine Verbindung 5 auf eine Säule 6, die einen Temperiermantel 7 aufweist, der mit einem Thermostaten 8 verbunden ist. Die Säule 6 ist mit einem Ionenaustauschergel 9 gefüllt. Der Auslaß 10 der Säule 6 ist mit einem Durchflußpolarimeter 11 verbunden, dessen Auslaß 12 über eine Leitung 13 an einem Sammelgefäß 14 endet, in dem die Lösung 15 mit dem racemisierten Hydantoin aufgefangen wird.

Die Vorlage 1 kann kontinuierlich oder diskontinuierlich mit der anfallenden Lösung 2 beschickt werden. Zur weiteren Kontrolle kann nach der Säule 6 bzw. nach dem Polarimeter 11 auch ein Autosampler für z. B. HPLC-Messungen zwischengeschaltet werden. Die anfallende Lösung 15 kann, sofern gewünscht auch in einem Fraktionssammler gesammelt bzw. direkt einer weiteren Racematspaltung zugeführt werden.

In den folgenden Beispielen wurde - sofern nicht anders vermerkt - für die Säule 6 eine temperierbare Säule XK-16/10 von Pharmacia, Freiburg verwendet, die mit ca. 32 ml entgastem Anionaustauscher Q-Sepharose fast flow (Pharmacia) bei einer Flußrate von 2,0 ml/min gepackt und mit dem jeweiligen Puffersystem, das zur Herstellung der Substratlösung Anwendung findet, äquilibriert (ca. 200 ml) wurde.

Der Umsatz gibt den Prozentsatz des eingesetzten Hydantoin-Enantiomeren an, der racemisiert wurde, ein 50 %iger Umsatz bedeutet entsprechend ein Enantiomerenverhältnis von 3 : 1.

### Beispiel 1

Als Substratlösung wurde eine Lösung von 0,2 g L-5-Indolylmethylhydantoin in 1000 ml 0,05 M Glycin-Puffer (Na⁺, pH 8,5) eingesetzt. Die Säule wurde auf 37°C temperiert und die Substratlösung mit einer Flußrate von 0,6 ml/min über die Säule gepumpt. Bei dieser und allen geringeren Flußraten wurde das Hydantoin praktisch vollständig racemisiert (≥99 %).

### Beispiel 2

Es wurde wie in Beispiel 1 verfahren, mit dem Unterschied, daß anstelle der Q-Sepharose fast flow DEAE-Sepharose fast flow (Pharmacia) verwendet wurde. Äquilibriert wurde die Säule mit einem Tris/HCl-Puffer (0,02 M, pH 8.5). Bei einer Flußrate von 0,1 ml/min. wurde eine Racemisierung von 92 % ermittelt.

### Vergleichsbeispiel 1

Es wurde wie in Beispiel 2 verfahren, mit dem Unterschied, daß anstelle der DEAE-Sepharose fast flow Sephacryl S-300 (Pharmacia) verwendet wurde. Unter den gegebenen Bedingungen konnte keine Racemisierung beobachtet werden.

### Beispiel 3

Wie in Beispiel 1 beschrieben, wurde L-5-Indolylmethylhydantoin racemisiert, wobei für die Puffer- und Substratlösung ein Phosphat-Puffer (0,05 M, K⁺, pH 6,5) eingesetzt wurde. Bei einer Flußrate von 1,2 ml/min wurde eine Racemisierung von 6 % erreicht.

### Beispiel 4

Beispiel 3 wurde mit einem Phosphat-Puffer (K⁺) vom pH 7,5 wiederholt. Bei einer Ourchflußrate von 0,6 ml/min wurde eine Racemisierung von 42 % erreicht.

### Beispiel 5

Beispiel 4 wurde wiederholt mit einem Phosphat-Puffer (K⁺) von pH 8,5. Allein durch die Variation des Puffermediums gegenüber Beispiel 1 veränderte sich der Umsatz, unter den gegebenen Bedingungen wurde eine Racemisierung von 64 % erzielt.

### Beispiel 6

Beispiel 1 bzw. 5 wurde dahingehend variiert, daß als Puffer- und Substratlösung 0,02 M Tris/HCl-Puffer (pH 8,5) eingesetzt wurde. Der Umsatz der Racemisierung betrug bei der Flußrate 0,6 ml/min 66 %.

### Beispiel 7

Es wurde wie in Beispiel 5 verfahren, mit dem Unterschied, daß als Substratlösung eine Lösung von 1,0 g D-5-Methylthioethylhydantoin in 1000 ml des Phosphat-Puffers eingesetzt wurde. Bei einer Flußrate von 0,3 ml/min. findet die Racemisierung des Substrates mit einem Umsatz von 66 % statt.

### Beispiel 8

Es wurde verfahren wie in Beispiel 6, mit dem Unterschied, daß eine Lösung von 1,0 g L-5-Carboxyethylhydantoin in 1000 ml des Puffers verwendet wurde. Bei einer Flußrate von 0,3 ml/min und geringer tritt eine vollständige Racemisierung des Substrates ein.

### Beispiel 9

Das Beispiel 8 wurde dahingehend variiert, daß L-5-Isopropylhydantoin verwendet wurde. Es wurde eine Racemisierung von nur 10 % erreicht.

### Beispiel 10

Das Beispiel 9 wurde bei einer Säulentemperatur von 70°C wiederholt bei sonst gleichgebliebenen Bedingungen. Bei dieser und allen höheren Temperaturen tritt eine vollständige Racemisierung des Substrates ein.

### Beispiel 11

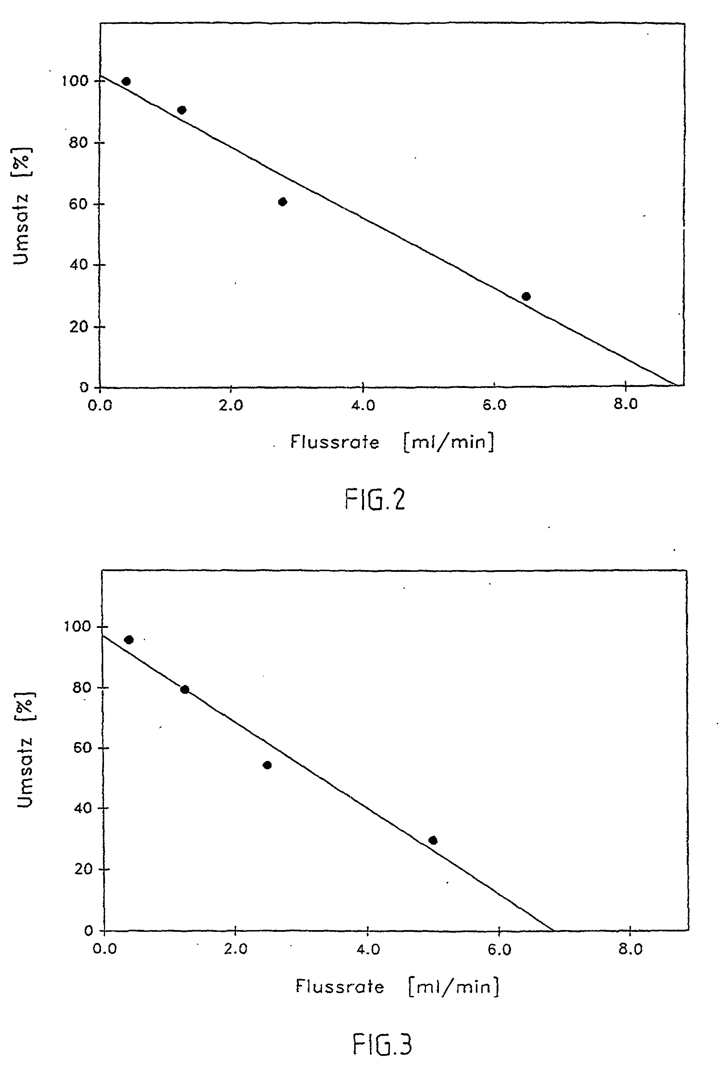

Mit diesem Beispiel wurde die Abhängigkeit des Umsatzes von der Flußrate untersucht, das Ergebnis ist in Fig. 2 graphisch abgebildet, es besteht ein linearer Zusammenhang zwischen der Flußrate und dem Umsatz.

Als Substratlösung wurde eine Lösung von 0,2 g D-5-Indolylmethylhydantoin in 1000 ml 0,05 M Glycin-Puffer (Na⁺, pH 8.5) eingesetzt. Die Säule (16 ml Q-Sepharose) wurde auf 37°C temperiert und die Substratlösung mit einer Flußrate von 6,5 ml/min über die Säule gepumpt. Bei dieser Flußrate wurde eine Racemisierung von 29,6 % erreicht. Sei einer Flußrate von 2,8 ml/min, 1,25 ml/min bzw. 0,4 ml/min wurde eine Racemisierung von 60,8 %, 90,3 % bzw. ∼ 100 % erreicht.

### Beispiel 12

Mit diesem Beispiel wurde die Eignung eines technischen Anionenaustauschers untersucht. Wie Fig. 3 zeigt, besteht auch hier ein linearer Zusammenhang zwischen Flußrate und Racemisierungsgeschwindigkeit (Umsatz). Das Säulenmaterial (hydrophobe Polystyrolmatrix) adsorbiert in der Äquilibrierungsphase aber zunächst deutlich mehr IMH. als die oben verwendete Q-Sepharose (hydrophile Sepharosematrix).

Es wurde wie in Beispiel 11 verfahren, mit dem Unterschied, daß anstelle der Q-Sepharose Amberlite IRA-410 (analytical grade, Serva, Heidelberg, Gelvolumen 5,3 ml) verwendet wurde. Bei einer Flußrate von 5,0 ml/min wurde eine Racemisierung von 29,7 % ermittelt. Bei einer Flußrate von 2,5 ml/min, 1,25 ml/min bzw. 0,4 ml/min wurde eine Racemisierung von 54,4 %, 79,4 % bzw. 96 % erreicht.

### Vergleichsbeispiel 2

Bei einem pH-Wert von 8,5 findet an dem stark sauren Kationenaustauscher Amberlite IR-122 analytical grade (16 ml Gelvolumen, Serva, Heidelberg) weder bei 3,0 ml/min noch bei 0,3 ml/min eine Racemisierung statt. Die Vergleichsbeispiele zeigen, daß Anionenaustauschergruppen für die Reaktion notwendig sind.

### Beispiel 13

Unter Verwendung von tertiären bzw. quartären Ammonium-Verbindungen wird die Racemisierung von D-5-IMH an flüssigen Anionenaustauschern untersucht.

In einer auf 37°C temperierten Polarimeterküvette (5 ml Volumen) wird die Racemisierung von D-5-IMH on-line verfolgt. 4 ml Substratlösung (0,2 g D-5-IMH in 1000 ml 0,05 M Glycin-Puffer (Na⁺. pH 8,5)) werden mit 1 ml H₂O dest. (Slindansatz) bzw. einer wässrigen Lösung einer tertiären (2) oder quartären Ammoniumverbindung (3, 4) (doppelter Ansatz) versetzt, die mit NaOH auf einen pH von ebenfalls 8,5 eingestellt wurde. Die Racemisierung wurde über mindestens 24 h verfolgt. Als Zusätze wurden getestet:
1. H₂O dest. (Blindansatz)
2. Triethanolamin (0,5 M)
3. Hexadecyltrimethylammoniumbromid (0,05 M) ("Cetyltrimethylammoniumbromid")
4. Tetrabutylammoniumhydrogensulfat (0,5 M)

Während Triethanolamin keinen (1,2 %/h) und Tetrabutylammoniumhydrogensulfat einen geringfügig (1,8 %/h) schnelleren Umsatz bewirken als der Blindwert (1,2 %/h), zeigt Hexadecyltrimethylammoniumbromid schon bei einem Zehntel der Konzentration, der übrigen Substanzen, eine schnellere Racemisierung (4,3 %/h).

Zu erklären ist der Unterschied zwischen den beiden quartären Ammoniumverbindungen (Hexadecyltrimethylammoniumbromid und Tetrabutylammoniumhydrogensulfat) möglicherweise mit einer stärkeren Abschirmung der positiven Ladung in der symmetrischen Verbindung.

## Patentansprüche

1. Verfahren zur Racemisierung von 5-monosubstituierten Hydantoinen,
**dadurch gekennzeichnet**,
daß man das zu racemisierende Hydantoin in einem Lösungsmittel bei einem pH im Bereich 6 - 13,5 mit einem Anionenaustauscher in Kontakt bringt.

2. Verfahren nach Anspruch 1,
**dadurch gekennzeichnet,**
daß ein organischer Anionenaustauscher verwendet wird.

3. Verfahren nach Anspruch 2,
**dadurch gekennzeichnet**,
daß ein Ionenaustauscher mit quartären Ammoniumgruppen oder vom DEAE-Typ verwendet wird.

4. Verfahren nach einem der vorhergehenden Ansprüche,
**dadurch gekennzeichnet,**
daß die Umsetzungstemperatur im Bereich von 0 - 100 °C liegt.

5. Verfahren nach einem der vorhergehenden Ansprüche,
**dadurch gekennzeichnet,**
daß das Lösungsmittel ein Puffersystem enthält.

6. Verfahren nach Anspruch 5,
**dadurch gekennzeichnet,**
daß das Verfahren in einem Glycin-Puffer durchgeführt wird.

7. Verfahren nach einem der vorhergehenden Ansprüche,
**dadurch gekennzeichnet,**
daß das zu racemisierende Hydantoin in einer Konzentration zwischen 0,1 - 250 g/l eingesetzt wird.

## Claims

1. A process for racemising 5-monosubstituted hydantoins,
characterised in that
the hydantoin to be racemised is brought into contact with an anion exchanger in a solvent at a pH in the range 6 - 13.5.

2. A process according to Claim 1,
characterised in that
an organic anion exchanger is used.

3. A process according to Claim 2,
characterised in that
an ion exchanger with quaternary ammonium groups or of the DEAE type is used.

4. A process according to one of the preceding Claims,
characterised in that
the conversion temperature is in the range 0 - 100°C.

5. A process according to one of the preceding Claims,
characterised in that
the solvent contains a buffer system.

6. A process according to Claim 5,
characterised in that
the process is performed in a glycine buffer.

7. A process according to one of the preceding Claims,
characterised in that
the hydantoin to be racemised is used at a concentration between 0.1 and 250 g/l.

## Revendications

1. Procédé de racémisation d'hydantoïnes 5-monosubstituées,
caractérisé en ce qu'
on met en contact l'hydantoïne à racémiser dans un solvant à un pH compris entre 6 et 13,5 avec un échangeur d'anions.

2. Procédé selon la revendication 1,
caractérisé en ce qu'
on utilise un échangeur d'anions organique.

3. Procédé selon la revendication 2,
caractérisé en ce qu'
on utilise un échangeur d'anions comportant des groupes ammonium quaternaire ou de type DEAE.

4. Procédé selon l'une quelconque des revendications précédentes,
caractérisé en ce que
la température de la réaction se situe dans un intervalle de 0 à 100°C.

5. Procédé selon l'une quelconque des revendications précédentes,
caractérisé en ce que
le solvant contient un système tampon.

6. Procédé selon la revendication 5,
caractérisé en ce que
le procédé est réalisé dans un tampon de glycine.

7. Procédé selon l'une quelconque des revendications précédentes,
caractérisé en ce que
l'hydantoïne à racémiser est utilisée à une concentration comprise entre 0,1 et 250 g/l.
